# FASCICULE DE BREVET EUROPEEN

(11) **EP 4 027 863 B1**
(45) Date de publication et mention de la délivrance du brevet: **06.08.2025**
(21) Numéro de dépôt: 20781048.2
(22) Date de dépôt: 11.09.2020
(51) Int. Cl.: A61B 3/10

(54) **DISPOSITIF ET PROCEDE DE DETECTION DE RUPTURE DE FILM LACRYMAL**
VORRICHTUNG UND VERFAHREN ZUR DETEKTION EINES TRÄNENFILMRISSES
DEVICE AND METHOD FOR DETECTING TEAR FILM BREAKUP

(30) Priorité: 13.09.2019 FR 1910129
(43) Date de publication de la demande: 20.07.2022
(73) Titulaire: E-Swin Developpement, 78550 Houdan (FR)
(72) Inventeur: BROTTIER, Yves-Vincent, 78113 ADAINVILLE (FR); OBIN, Arnaud, 78660 PARAY-DOUAVILLE (FR); PERRIN, Nelson, 78730 SAINTE-MESME (FR)
(74) Mandataire: Plasseraud IP
(86) Numéro de dépôt international: PCT/FR2020/051581
(87) Numéro de publication internationale: WO 2021/048511

(56) Documents cités:
- EP-A1- 1 844 702
- WO-A1-2017/167939
- WO-A1-2018/156022
- JP-A- 2000 254 099
- US-A- 5 159 361
- US-A1- 2010 253 907
- US-A1- 2014 104 574

## Description

### Domaine technique

L'invention relève du domaine de la détection du syndrome de sécheresse oculaire.

### Technique antérieure

Les tests d'évaluation du syndrome de sécheresse oculaire comprennent un test dans lequel les praticiens utilisent de la fluorescéine et regardent directement l'évolution du film lacrymal d'un patient. Ce test dit invasif donne en général un temps d'apparition d'une première rupture de film lacrymal.

Il existe aussi un test non invasif de temps de rupture (« Non Invasive Breakup Time » en anglais) qui évalue la qualité du film lacrymal et qui permet de déterminer le temps entre un clignement de paupière et une rupture du film lacrymal et qui utilise la réflexion sur la cornée d'une mire pourvue d'un motif constitué de cercles concentriques et de lignes radiales.

Lorsqu'une rupture du film apparaît, le motif réfléchi présente des déformations des lignes et/ou des cercles ou des zones manquantes.

Les images du motif réfléchi sont acquises en séquences par une caméra et traitées par un système de traitement informatisé qui analyse l'évolution du motif dans le temps pour détecter les déformations du motif et en déduire le temps d'apparition des zones de rupture du film.

Les dispositifs actuellement disponibles utilisent souvent des motifs constitués de cercles concentriques et de lignes radiales. Ceci occasionne des problèmes de plusieurs ordres :

compte tenu de la forme des mires, et de la couverture de la cornée par secteurs de disque, la résolution spatiale est intrinsèquement moins bonne sur les bords du champ qu'au centre.

Au centre du champ, là où les lignes radiales convergent, il est difficile de déterminer la déformation d'un motif.

L'augmentation de la résolution spatiale nécessiterait de resserrer le motif de la mire. Or, les algorithmes de détection de déformations de cercles qui requièrent de trouver les cercles les plus probables dans l'image, d'évaluer si un point de l'image appartient à un cercle probable et de calculer la distance d'un point donné au cercle auquel il est censé appartenir sont complexes et le temps de traitement et de calcul pour détecter les zones de rupture à partir de l'image est important.

Il est donc clair que : d'une part les dispositifs présentant le motif classique de cercles concentriques et lignes radiales souffrent d'un défaut d'homogénéité de la résolution spatiale, d'autre part augmenter la résolution en bord de champ est pénalisant sur les temps de calcul.

Du fait de la faible résolution de ce procédé, la mesure peut donner des valeurs d'apparition de ruptures imprécises et surévaluées.

Par ailleurs, dans l'art antérieur le document WO 2018/156022 A1 traite d'un dispositif de détermination de l'état de la couche lacrymale de l'œil, comprenant une pluralité de points sources lumineux pour projeter une pluralité de rayons lumineux sur une surface de cornée ; un système lentille-caméra agencé pour recevoir des rayons lumineux réfléchis sur la surface de la cornée, formant ainsi un motif de points d'image et une unité de calcul agencée pour fournir les données représentatives de l'état de la couche lacrymale de l'œil à un utilisateur et le document US 2010/253907 A1décrit un dispositif de mesure de film oculaire comportant un dispositif d'éclairage comportant une pluralité de DELs blanches adressables indépendamment pour générer sur l'œil une image carrelée. Les documents EP 1 844 702 A1, WO 2017/167939 A1, US 2014/104574 A1 traitent d'autres dispositifs connus.

JP 2000-254099 divulgue un appareil ophtalmologique pour détecter automatiquement les ruptures du film lacrymal (paragraphe [0001]). L'appareil comprend un moyen de projection qui projette un motif prédéterminé sur la cornée (paragraphes [0010], [0012]), une plaque de projection de motif 6 comportant des bandes claires et sombres alternées (paragraphe [0012]), une caméra (11) qui capture l'image réfléchie du motif (paragraphe [0013]), et un circuit électrique (15) qui détecte les changements d'état dans l'image réfléchie dus au dessèchement des larmes (paragraphes [0015]-[0017]).

### Problème technique

Ainsi il existe une réelle incertitude sur l'apparition du phénomène de rupture du film oculaire et en outre, les algorithmes de détection de déformations de cercles qui requièrent de trouver les cercles les plus probables dans l'image, d'évaluer si un point de l'image appartient à un cercle probable et de calculer la distance d'un point donné au cercle auquel il est censé appartenir sont complexes et le temps de traitement et de calcul pour détecter les zones de rupture à partir de l'image est important. En outre, comme vu plus haut la précision de mesure est faible et les machines utilisant ce principe ne sont pas assez sensibles pour mesurer des ruptures peu étendues notamment du fait que la résolution diminue lorsque l'on s'écarte du centre du motif à cercles et rayons. Or le praticien a besoin d'une mesure fiable et reproductible pour effectuer son diagnostic.

### Exposé de l'invention

L'invention vient améliorer la situation et propose en premier lieu d'utiliser un dispositif comportant un motif constitué à partir de lignes claires et sombres et d'observer la réflexion de ce motif sur la cornée.

Pour ce faire l'invention propose d'une part un dispositif de détection d'une ou plusieurs ruptures d'un film lacrymal, ce dispositif étant défini à la revendication 1 et comportant une plaque translucide rétroéclairée munie d'une mire se positionnant devant au moins un œil d'un patient et pourvue d'un motif se réfléchissant sur l'œil du patient, au moins une caméra photographique numérique reliée à un système de calcul pourvu de moyens de traitement et d'analyse d'images, un objectif de la caméra pointant vers l'œil du patient afin de photographier une réflexion du motif de la mire sur l'œil du patient, pour lequel le motif de la mire est pourvu d'une série de lignes sous forme d'une alternance de lignes transparentes et de lignes opaques horizontales ou verticales rétroéclairées pour former des lignes claires et sombres réfléchies sur l'œil du patient et pour lequel les moyens de traitement et d'analyse d'images sont configurés pour détecter des déformations desdites lignes claires ou sombres défléchies sur l'œil du patient et identifier les ruptures de film lacrymal révélées par ces déformations.

Le dispositif de la présente invention basé sur la détection de lignes claires ou sombres dans une alternance de lignes claires et sombres évite les problématiques des dispositifs à mires à motifs circulaires et permet une détection fine des ruptures de film, bien qu'il paraisse de prime abord inadapté à un œil qui comporte une courbure sphérique.

En outre, du fait que la résolution du motif reste homogène sur toute la zone mesurée, la présente invention permet de procéder à une série de mesure retraçant l'évolution de la croissance des ruptures du film lacrymal.

Les caractéristiques du dispositif exposées dans les paragraphes suivants peuvent, optionnellement, être mises en œuvre. Elles peuvent être mises en œuvre indépendamment les unes des autres ou en combinaison les unes avec les autres :

Les lignes de la série de lignes sont préférablement des lignes parallèles.

La largeur des lignes est avantageusement croissante depuis une ligne médiane de la mire vers des bords de la mire.

la mire est pourvue d'une courbure cylindrique selon une génératrice verticale.

La mire est avantageusement de dimensions suffisantes pour produire une image sur une majeure partie de la cornée de l'œil ou des yeux du patient, par exemple elle est conçue pour englober une majeure partie du champ oculaire humain.

Les moyens de traitement et d'analyse d'image peuvent avantageusement comporter des moyens de conversion de l'image en niveaux de gris.

Les moyens de traitement et d'analyse d'images peuvent avantageusement comporter des moyens de filtrage passe-bande anisotropes.

Les moyens de traitement et d'analyse d'images peuvent avantageusement comporter des moyens d'analyse de l'image selon des successions de pixels perpendiculaires à la direction des lignes, des moyens de recherche de segments lumineux ou sombres dans lesdites successions de pixels, des moyens de quantification de la taille desdits segments lumineux ou sombres et d'élimination des segments dont la taille est incompatible avec une image des lignes du motif.

Les moyens de traitement et d'analyse d'images peuvent avantageusement comporter des moyens de marquage/catalogage d'objets segments lumineux ou sombres adaptés à réaliser une reconstruction de premiers objets correspondant à des branches de lignes claires ou sombres du motif et à réaliser une élimination de seconds objets de forme non compatible avec lesdites lignes claires ou sombres.

Les moyens de traitement et d'analyse d'images peuvent avantageusement comporter des moyens de calcul de raccordement de branches de lignes claires ou sombre sur un même axe, des moyens de calcul d'une régression polynomiale sur les données de lignes claires ou sombres en sorte de calculer des courbes RMS représentatives de bords desdites lignes et des moyens de calcul de détection de zones de rupture du film lacrymal sur des points d'image des bords de lignes dont la distance avec ladite courbe est au-delà d'une valeur de tolérance donnée.

Selon un autre aspect de la présente demande qui peut être utilisé indépendamment de la mire décrite ci-dessus, le dispositif peut comporter des moyens de suivi de l'œil ou des yeux du patient basés sur une reconnaissance et un suivi d'iris.

Ces moyens permettent ici de recaler les ruptures de film lacrymal détectées par rapport à l'œil analysé mais pourraient être utilisé dans d'autres contextes où un suivi de l'iris est souhaité.

Selon un aspect particulier du dispositif de la présente demande, la plaque translucide rétroéclairée porteuse de la mire et la ou les caméras sont intégrées dans un appareil de mesure ophtalmique tel qu'un bâti ophtalmologique muni d'un support d'appui de la tête du patient ou intégrées dans un casque porté par le patient.

La présente demande concerne en outre un procédé de détection de ruptures de film lacrymal au moyen d'un dispositif ci-dessus qui comporte une détection de clignement de paupière fournissant une origine des temps et au moins une séquence comportant une succession de prises d'images et de calcul de zones de rupture à partir de l'origine des temps et jusqu'à un clignement de paupière suivant.

Les caractéristiques du procédé exposées dans les paragraphes suivants peuvent, optionnellement, être mises en œuvre. Elles peuvent être mises en œuvre indépendamment les unes des autres ou en combinaison les unes avec les autres :

Le procédé peut comporter une prise d'image toutes les 0,2 à 0,5 secondes et préférablement toutes les 0,3 secondes.

Le procédé comporte avantageusement pour chaque image prise, une succession d'étapes de traitement et d'analyse comportant
- une conversion de l'image en niveaux de gris;
- un filtrage de l'image convertie au moyen d'un filtre passe-bande anisotrope afin de réduire le vignettage et d'homogénéiser la luminosité de l'image ;
- une recherche de segments lumineux ou sombres colonne par colonne dans l'image, une étape de quantification de la taille desdits segments lumineux ou sombres et une étape d'élimination des segments dont la taille est incompatible avec une correspondance avec des lignes du motif ;
- une étape de marquage/catalogage d'objets segments lumineux ou segments sombres, de reconstruction de premiers desdits objets correspondant à des objets branches de lignes du motif et une étape d'élimination de seconds desdits objets de forme non compatible avec lesdites lignes du motif ;
- une étape de raccordement de branches de lignes de même niveau et une étape de calcul d'une régression polynomiale sur les données de lignes en sorte de calculer une courbe RMS des bords de lignes ;
- un calcul de zones de rupture du film lacrymal par calcul de la distance des points de bords de lignes à ladite courbe, lesdites zones de rupture correspondant aux bords de lignes dont la distance à ladite courbe est supérieure à une valeur de tolérance donnée.

Dans ce procédé, le filtrage anisotrope et la régression polynomiale sont des moyens de traitement d'image qui concourent à un traitement rapide des images et une bonne détection des défauts occasionnés par les ruptures de film lacrymal.

Selon un aspect de la demande pouvant être utilisé dans un procédé autre, le procédé comporte des étapes de suivi de l'œil ou des yeux du patient au moyen d'un procédé de suivi d'iris. Ces étapes permettent ici de de repositionner les zones de rupture de film lacrymal détectées par rapport à l'œil analysé.

Les étapes de suivi de l'œil peuvent comporter :
- une première transformation de l'image par l'application d'un filtre passe bande anisotrope appliqué dans la direction de la largeur de l'œil pour produire des paires de transition montantes sombre vers clair et descendantes clair vers sombre selon un axe horizontal de l'œil ;
- une segmentation de l'image pour rechercher les paires de transitions montante et descendante qui forment des segments devant être représentatifs de zones lumineuses dans l'image ;
- un filtrage de l'image qui élimine les segments clairs de la zone centrale comportant le motif et des zones supérieure et inférieure de l'image ;
- la prise en compte des segments clairs, les autres zones de l'image n'étant plus considérées dans cette analyse et un premier calcul d'un cercle RMS du tour de l'iris à partir des extrémités droites des segments clairs à gauche de l'image et des extrémités gauches des segments clairs à droite de l'image;
- une étape de suppression des points trop éloignés du cercle RMS ;
- pour les points restants, une nouvelle étape de calcul de cercle RMS (93) pour suivre le contour de l'iris.

La demande propose en outre un programme informatique comportant des instructions pour la mise en œuvre du procédé de détection des ruptures et du procédé de suivi de l'œil lorsque ce programme est exécuté par un processeur.

Ce programme peut être stocké dans un support d'enregistrement non transitoire, lisible par un ordinateur, sur lequel est enregistré un programme pour la mise en œuvre du procédé lorsque ce programme est exécuté par un processeur.

La demande propose enfin une mire pour la mise en œuvre du dispositif de l'invention qui est réalisée au moyen d'un film polymère transparent pourvu de lignes opaques imprimées ou sérigraphiées sur ledit film. La mire peut comporter au moins une zone évidée entouré d'un cadre opaque ou des zones de substrat transparentes dans une zone médiane de la mire.

### Brève description des dessins

D'autres caractéristiques, détails et avantages de l'invention apparaîtront à la lecture de la description détaillée ci-après, et à l'analyse des dessins annexés, sur lesquels :
[Fig. 1] est une vue à plat de face d'une mire pourvue d'un motif de l'invention ;
[Fig. 2] est une vue schématique d'un dispositif de la présente demande;
[Fig. 3] montre un premier exemple de support de mesure utilisable dans le cadre de la présente demande ;
[Fig. 4] représente une image d'un œil d'un patient suite à une première étape de traitement ;
[Fig. 5A], [Fig. 5B], [Fig. 5C], [Fig. 5D], [Fig. 5E] représentent diverses étapes de traitement de l'image de l'œil de la figure 4;
[Fig. 6A], [Fig. 6B] représentent des détails de la figure 5E;
[Fig. 7] représente une image de l'œil de la figure 4 après analyse d'image ;
[Fig. 8] représente une image d'un œil d'un patient qui regarde vers une caméra ;
[Fig. 9A], [Fig. 9B], [Fig. 9C] représentent des étapes de détermination de la position de l'iris de l'œil de la figure 8 selon un aspect de la demande;
[Fig. 10] représente une image d'un œil d'un patient dont le regard s'écarte d'une caméra ;
[Fig. 11A], [Fig. 11B], [Fig. 11C] représentent des étapes de détermination de la position de l'iris de l'œil de la figure 10 selon un aspect de la demande;
[Fig. 12] schématise un procédé de détection de zones de rupture de film lacrymal;
[Fig. 13] schématise un procédé de repositionnement de zones de rupture de film lacrymal dans un référentiel lié à l'œil ;

### Description des modes de réalisation

Les dessins et la description ci-après décrivent un ou plusieurs exemples de réalisation qui pourront donc non seulement servir à mieux faire comprendre les objets de la présente demande, mais aussi contribuer à sa définition, le cas échéant.

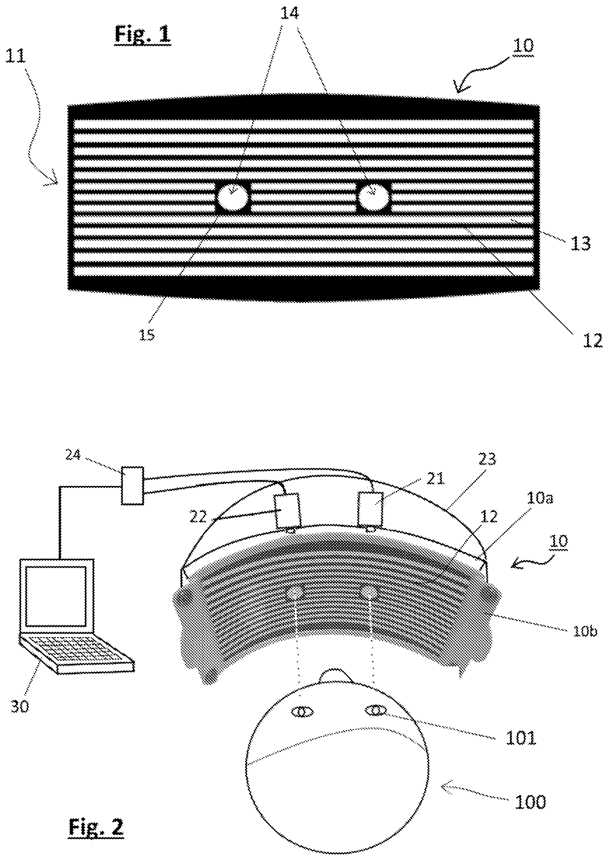

Le procédé et le dispositif de détection de ruptures de film lacrymal selon la présente demande utilisent une mire 10 représentée en figure 1 et réalisée à partir d'un film polymère transparent comportant un motif 11 réalisé avec une alternance de lignes 12, 13 droites et parallèles. Le motif comporte des lignes opaques 12, par exemple des lignes noires séparées par des lignes transparentes 13 laissant passer la lumière d'une source de lumière traversant un support translucide derrière le motif pour réaliser des lignes claires de sorte que les lignes claires du motif se reflète sur l'œil ou les yeux d'un patient.

Selon l'exemple, le motif 11 de la mire 10 comporte douze lignes opaques 12 sans compter les bordures supérieure et inférieure de la mire. Ces lignes opaques sont séparées par des lignes transparentes 13 et centrées autour d'une ligne translucide médiane. La mire peut être montée sur un cadre plastique pour la rendre plus aisée à manipuler.

Par convention, on appellera axe horizontal un axe parallèle à un axe passant par les yeux du patient et axe vertical l'axe perpendiculaire à cet axe et dans l'exemple illustré, les lignes du motif sont horizontales.

Comme représenté en figure 2, la mire 10 sur son support plastique 10b est positionnée sur un support translucide 10a lui-même percé de trous de passage des objectifs caméras et le dispositif utilise une source de lumière diffuse 23 derrière le support du motif pour illuminer le motif. La réflexion du motif est observée par une ou deux caméras numériques et, pour observer les deux yeux, deux caméras numériques 21, 22 sont prévues.

La source de lumière diffuse 23 peut être réalisée au moyen d'une boîte ou sphère d'intégration ou de manière similaire à un rétroéclairage d'écran LCD par exemple.

La mire est pourvue de deux zones évidées 14 centrées dans des cadres opaques 15 sur une ligne médiane horizontale. Les zones évidées sont distantes d'une distance correspondant à un écart oculaire moyen comme représenté en figure 1. De retour à la figure 2, les caméras sont positionnées derrière les zones évidées et se retrouvent en face des yeux 101 du patient 100. Les objectifs des caméras filment ou photographient les yeux du patient au travers des zones évidées. Les zones évidées peuvent être remplacées par des zones de substrat transparentes de la mire si les propriétés optiques du substrat de la mire et son niveau de propreté sont compatibles avec la formation d'image (transparent et non diffusant).

Les caméras sont par exemple des caméras de type CMOS à capteur 1/4". Selon l'exemple non limitatif représenté, les zones transparentes sont des trous circulaires d'un diamètre adapté aux objectifs des caméras. Pour des caméras avec des optiques de focale de l'ordre de 4mm, des trous de l'ordre de 14 mm sont réalisés et les cadres opaques sont des carrés opaques de l'ordre de 16mm x 16mm. Ces cadres ont pour but de précisément terminer les lignes en amont des trous recevant les objectifs des caméras.

Dans l'exemple de réalisation, la ou les caméras fournissent des images avec une définition de 1920x1080 qui suffit pour une analyse de rupture de film sans accroître la charge de calcul du système.

Les signaux vidéo ou des caméras sont envoyés vers un dispositif de traitement informatisé 30 ou système de calcul, interne ou externe au dispositif de mesure et, pour éviter de dupliquer ce dispositif de traitement, les signaux vidéo des deux caméras passent par un multiplexeur, sur une carte électronique 24 du dispositif, le multiplexeur permettant d'envoyer l'un ou l'autre des canaux vidéo au choix vers le dispositif de traitement 30.

Les caméras filment ou photographient l'image des lignes du motif réfléchie dans la cornée du patient. Comme la cornée en première approche peut être considérée comme un dioptre sphérique, elle présente une importante courbure de champ et l'image présente une importante distorsion. Afin d'au moins partiellement compenser la distorsion et de conserver sur les images capturées des lignes dont la largeur varie peu depuis l'axe médian du motif vers le bord de l'image, le motif de la mire comporte des lignes avec une période croissante depuis l'axe central du motif vers les bords du support parallèles aux lignes. Par exemple, une ligne blanche centrale peut avoir une hauteur d'environ 2,8 mm, les lignes noires adjacentes une hauteur de 2,2 mm alors que les dernières lignes blanches ont une hauteur de 3,8 mm et les lignes noires précédant ces lignes blanches ont une hauteur de 2,8 mm, la progression étant optimisée pour compenser la courbure d'un œil standard. Dans le procédé décrit ce sont les lignes claires qui sont exploitées pour rechercher les zones de ruptures. Le nombre et la largeur des lignes claires peut être différent des exemples donnés mais est choisi pour obtenir une définition suffisante pour une détection significative des zones de rupture de film en fonction de la résolution de la ou des caméras.

Dans le principe de la présente demande, les lignes blanches sont reflétées par le dioptre cornéen et ressortent alors qu'elles sont rétrodiffusées dans la conjonctive bulbaire de l'œil et l'iris où l'alternance lignes sombres lignes claires réalise plutôt un fond continu plus ou moins lumineux dépendant du ratio entre les surfaces transparentes de la mire et sa surface totale.

En figure 2, la mire vue de dessus est courbée autour d'un axe vertical pour former une portion de cylindre et suivre la courbure de la tête du patient 100 de sorte que l'image de la mire couvre une grande partie de la cornée.

Les raisons en sont :
a. La conjugaison optique :
   La caméra voit le reflet de la mire par la cornée considérée en première approche comme un miroir sphérique de rayon 8 mm environ, soit 4 mm de focale. Compte tenu de cette courte focale, il faut que la mire (l'objet dans la conjugaison optique) soit grande pour que la taille de l'image par la cornée soit suffisamment grande, de taille comparable au diamètre extérieur de l'iris. C'est ce qui détermine la taille du masque.
b. La photométrie :
   Pour que la mire soit visible, il faut que des rayons lumineux issus des bords extrêmes de la mire entrent dans la pupille de l'objectif. La cornée étant un miroir de forte courbure, il faut qu'en bord de champ les rayons lumineux arrivant sur la cornée soient rasant.

C'est ce qui justifie la courbure de la mire.

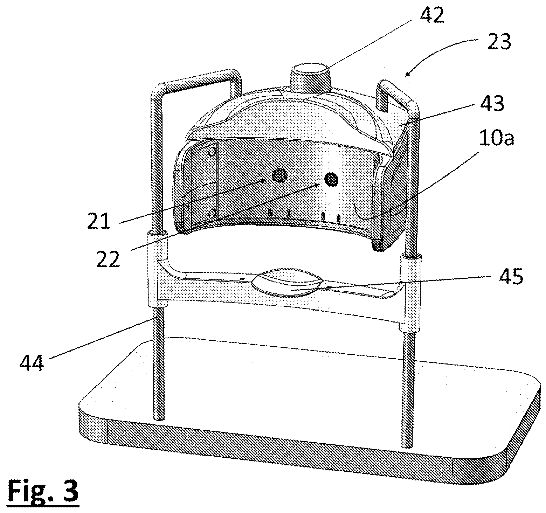

Selon la figure 3 le dispositif de mesure est monté sur un bâti ophtalmologique avec appui menton et front comportant des montants 44, un carter 43 qui intègre les caméras et qui reçoit la mire 10 sur une face courbe frontale devant laquelle va se placer le patient, menton en appui sur un support 45. Sur un tel bâti, les yeux du patient sont à une distance de la mire d'environ 50 mm pour une focale de caméra de 4mm. Le dispositif de mesure peut aussi être intégré à un casque porté par le patient.

La focale et la distance sont choisies pour permettre de de voir tout l'œil compte tenu des variations de positionnement de l'œil par rapport à la caméra (écart interoculaire différent d'un individu à l'autre). Ensuite on choisit une fenêtre d'intérêt centrée sur la pupille du patient, par action de l'opérateur qui pointe le centre de la pupille sur l'image.

De sorte que les lignes de la mire soient nettes sur l'image, la mise au point se règle avec une molette 42.

Le procédé de mesure de la présente demande a pour objet de détecter et de mesurer la croissance des zones de sécheresse oculaire et de rupture du film lacrymal. Il comporte une répétition de prises de vues de l'un ou des yeux du patient selon une fréquence de répétition de l'ordre de 0.2 à 0,5 secondes et en pratique de 0,3 secondes après un clignement des yeux ou de l'œil.

Le procédé est décrit principalement dans le cadre d'une mire à lignes horizontales c'est-à-dire selon un axe passant par les deux pupilles du patient mais est adaptable notamment moyennant une rotation de 90° des moyens de traitement de séries de pixels et du filtre passe bande anisotrope décrit ci-après. Par ailleurs le procédé décrit dans le cadre de la détection des lignes claires peut s'appliquer à une détection des lignes sombres.

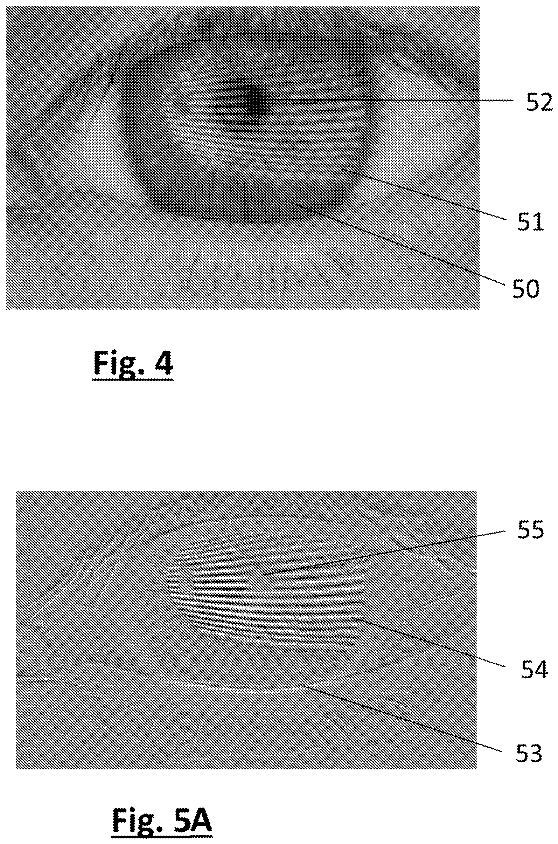

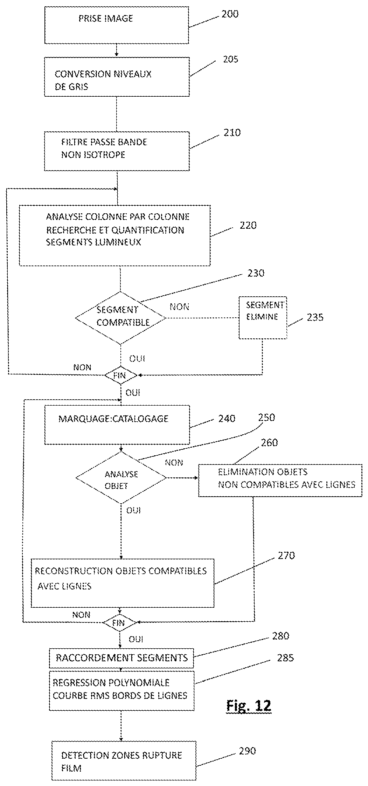

Les étapes de mesure dans le système de calcul 30 comprennent des étapes de traitement d'image qui, partant d'une capture de l'image originale de l'œil du patient comprennent;
- une conversion de l'image en niveaux de gris selon l'étape 205 de la figure 12 et dont un exemple de résultat est représenté en figure 4 où l'on distingue l'image 51 du motif sur l'iris 50 avec l'image du cadre 52 entourant l'objectif de la caméra. Sur cette image et l'image d'origine en couleurs, la forme des lignes claires réfléchies comporte des défauts locaux (notamment des bords de lignes irréguliers qui révèlent déjà des déformations du film lacrymal) ;
- Une deuxième étape 210 de la figure 12 comporte une application d'un filtre passe bande anisotrope appliqué selon une direction perpendiculaires à la direction des lignes reflétées, soit une direction verticale et donc sur les colonnes de pixels de l'image dans le cas d'une mire à lignes horizontales ou d'une mire à lignes verticales, l'image étant dans ce cas tournée de 90° pour obtenir des lignes claires orientées dans une direction horizontale, et configuré pour garder les transitions franches entre les niveaux de gris et supprimer ou atténuer les modulations de basse fréquence spatiale et de fréquence spatiale plus élevée dans la direction perpendiculaire. L'image en sortie de filtre est représentée à la figure 5A. Ce filtre a notamment pour utilité de s'affranchir des phénomènes de vignettage et des défauts d'homogénéité de l'éclairage. Cette transformation accentue les lignes de la paupière 53, les lignes claires 54 du motif de la mire et conserve l'image du cadre 55.

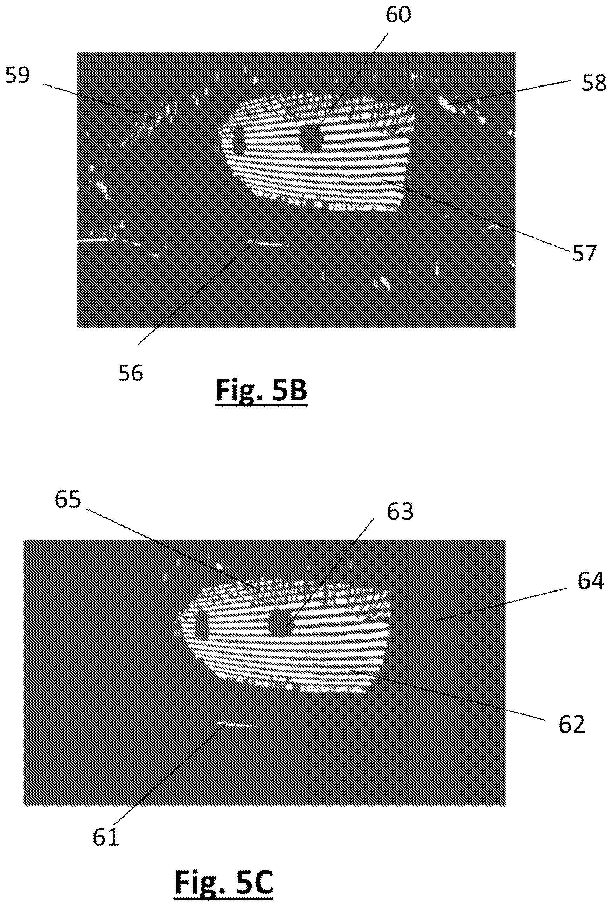

Ensuite, toujours dans le cas de lignes claires orientées dans une direction horizontale, le procédé comporte une analyse de l'image 220 par colonnes de pixels selon la figure 12 qui va rechercher des segments lumineux verticaux. L'image résultante comporte alors des lignes 56, 57, 58, 59 comme représenté en figure 5B. Une fois cette analyse faite, il est procédé à une qualification des segments lumineux par leur taille, étapes 230, 235. Ceci permet d'éliminer les segments trop grands ou trop courts qui ne correspondent clairement pas à des segments de lignes du motif, par exemple les segments faisant partie du contour des paupières. A l'issue de cette étape, la figure 5C représente l'image où subsistent les segments de colonnes constituant la ligne isolée 61, les lignes de l'image du motif 62, le fond 64 et le cadre 63. Il est à noter que les cils causent une fragmentation importante des lignes 65 du haut de l'image. Dans le cas d'une mire à lignes verticales, l'analyse et la qualification des segments se fait par lignes de pixels.

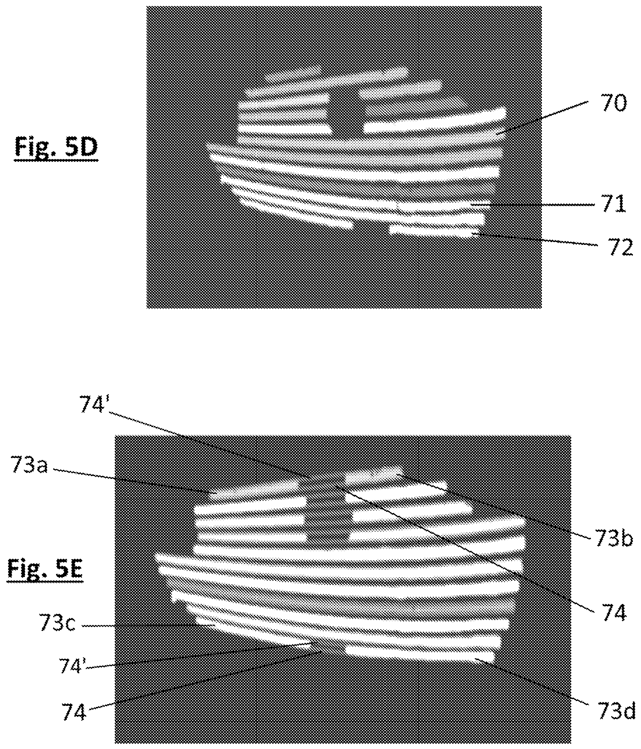

Lorsque la segmentation est terminée, le procédé de traitement comporte un algorithme de marquage/catalogage 240, 250, 260 des segments lumineux pour obtenir les objets représentatifs de branches lumineuses de lignes du motif et pour rejeter les objets lumineux n'ayant pas la forme recherchée qui sont alors considérés comme des artéfacts. Cet algorithme comporte en premier lieu un raccordement des segments de colonnes contigus pour reconstituer des branches horizontales. Le résultat de ce marquage/catalogage est représenté en figure 5D pour lequel à chaque ligne trouvée est affecté une couleur ici représentée en niveaux de gris. Ce catalogage permet de créer des lignes complètes 70 ou des branches isolées 71, 72.

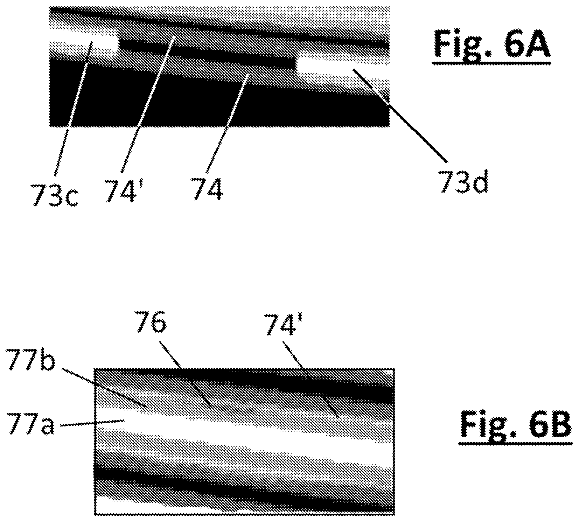

Une étape suivante est un raccordement 280 des branches de lignes lumineuses de même niveau (par exemple de largeur et d'altitude similaire) dans l'image puis une régression polynomiale 285 utilisant un polynôme d'ordre supérieur à deux en sorte de calculer une courbe RMS de la forme des bords de lignes. Cette étape est représentée en figure 5E. Dans cette figure on remarque notamment des raccordements par les courbes RMS inférieure 74 et supérieure 74' des branches 73a, 73b des lignes morcelées au niveau de l'image du cadre entourant l'objectif de la caméra et des branches 73c, 73d de la ligne inférieure. L'agrandissement de la figure 6A permet de mieux distinguer les courbes RMS 74, 74' entre les branches 73c, 73c du bas de l'image. Ensuite est réalisée une détection des sites ou zones de rupture du film 290 aux endroits où les bords des lignes comportent des points de mesure qui s'écartent de la forme donnée par le polynôme, avec deux critères :
- l'écart par rapport au polynôme est supérieur à un seuil,
- ce dépassement du seuil existe sur plusieurs colonnes contiguës.

Ceci est par exemple représenté en figure 6B dans la zone 76 où le bord de ligne 77b de la ligne 77a n'atteint pas la courbe 74'.

Comme vu plus haut, le procédé peut être basé sur un traitement des lignes sombres. Grouper les paires de transition (montante et descendante dans le cas des lignes claires) permet de faire un contrôle de cohérence sur la largeur du segment obtenu et de rejeter les segments trop larges ou trop étroits pour faire partie de l'image de la mire. Une fois qu'on a déterminé les branches, la régression polynomiale est effectué de chaque côté de la branche : un polynôme pour les transitions montantes, un polynôme pour les transitions descendantes. Le procédé de l'invention permet donc tout aussi bien chercher les segments sombres, les lignes sombres et arriver aux même régressions polynomiales et au même résultat final.

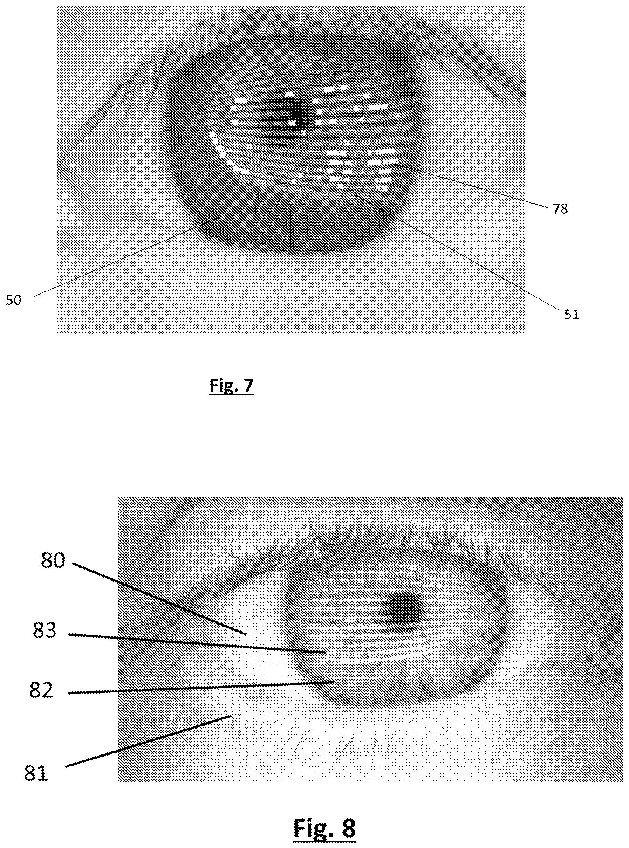

Le résultat des mesures est une cartographie des sites de rupture de film lacrymal représentée à la figure 7 pour laquelle la cartographie des points de rupture 78 est positionnée sur l'image initiale couleur de l'œil reprise ici en niveaux de gris.

Comme vu plus haut, les images sont capturées environ toutes les 0,3 secondes. Une origine des temps est définie comme un clignement de paupière et en répétant la mesure pour chaque image sur une période de temps permet la construction d'une carte des ruptures en fonction du temps.

Un problème à considérer est que le regard du patient peut changer de direction pendant la période d'acquisition d'images.

Comme la caméra observe les réflexions du motif sur la cornée qui se comporte en première approximation comme un dioptre sphérique, la position de l'image du motif reste quasiment invariante dans l'image délivrée par la caméra alors que la position de l'iris change si le patient tourne les yeux. De ce fait un point donné de l'image de la mire n'est pas lié à un point fixe de la cornée, mais au contraire à un point dépendant de la direction du regard. Ceci implique que la mesure doit être référencée par rapport à la position de l'œil observé et non par rapport à l'image du motif.

Pour ce faire il faut suivre la position de l'œil dans chaque image. Il a été préféré de suivre le contour extérieur de l'iris de l'œil du fait qu'un contraste important existe avec la conjonctive bulbaire, qui est de couleur claire et sur laquelle on n'a pas de réflexion de la mire alors que la pupille est plus délicate à suivre du fait du reflet de la mire qui complique l'analyse de l'image.

Le procédé qui suit peut être mis en œuvre dans le cadre de la présente demande ou indépendamment pour d'autres mesures sur les yeux. Ce procédé est par ailleurs indépendant de l'orientation des lignes de la mire.

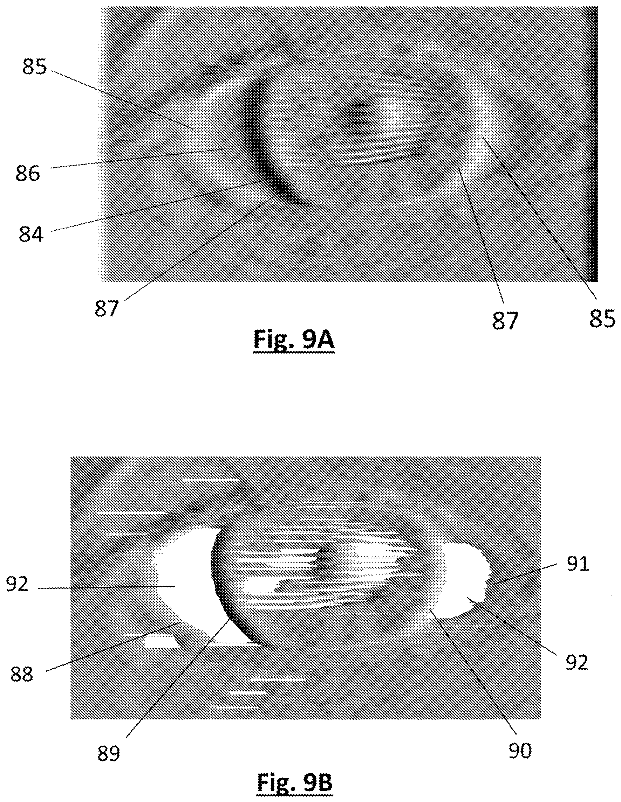

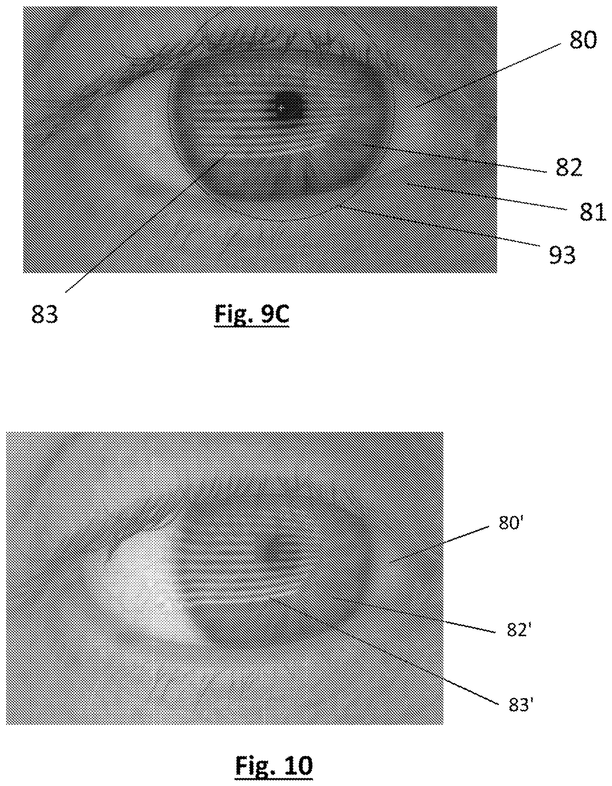

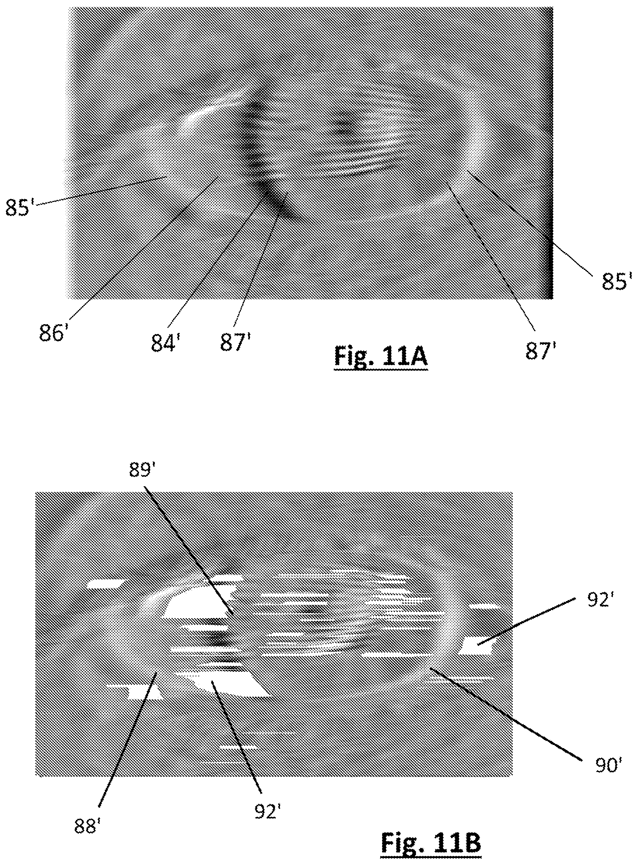

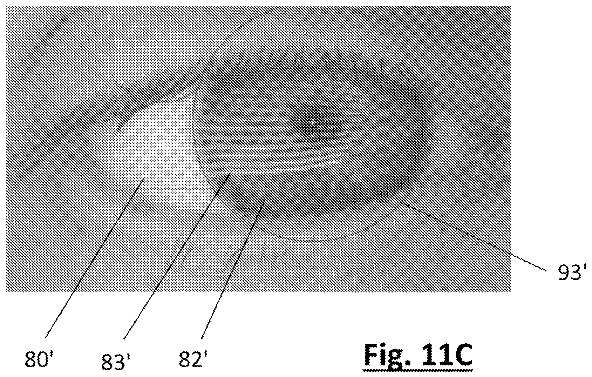

Les figures 8, 9A, 9B et 9C correspondent aux traitements d'image sur un œil d'un patient regardant la caméra et les figures 10, 11A, 11B et 11C correspondent aux traitements d'image sur un œil d'un patient dont le regard s'écarte de la caméra.

Selon la figure 8, l'œil 80 regarde droit devant, l'image du motif 83 est centrée par rapport à l'iris 82 lui-même centré par rapport à la paupière 81.

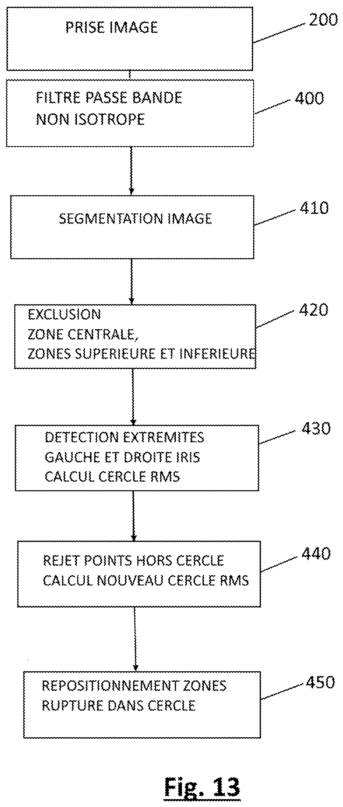

Le procédé de traitement des images pour retrouver la position de l'iris est schématisé à la figure 13. Il comporte une première transformation de l'image par l'application d'un filtre passe bande anisotrope 400 appliqué horizontalement pour détecter les transitions lumineuses selon un axe horizontal. Dans cette opération on recherche à distinguer les transitions descendantes (clair vers sombre) et les transitions montantes (sombre vers clair) et pour la compréhension, les transitions clair vers sombre (transition descendante) sont traduite par le croissant sombre 84 de la représentation en niveaux de gris de la figure 9A et les transitions sombre vers clair (transition montante) sont traduites par le croissant clair 85 de la figure 9A. Les parties de l'image sans transitions significatives deviennent gris moyen, comme le croissant 86 par exemple, et le contour de l'iris se matérialise par une portion d'anneau 87 qui se retrouve sur le côté gauche à côté d'une transition clair vers sombre et de l'autre côté de l'œil à côté d'une transition sombre vers clair.

De retour à la figure 13, une deuxième opération consiste en une segmentation de l'image 410 pour rechercher les paires de transitions montante et descendante, 84, 85 en figure 9A par exemple, qui forment des frontières des zones lumineuses dans l'image, notamment autour de la conjonctive bulbaire. Ces paires de transitions matérialisées par les limites 88, 89 et 90, 91 sur la figure 9B encadrent des zones 92 définissant potentiellement la conjonctive bulbaire.

A partir de cette transformation le procédé comporte selon la figure 13 un filtrage de l'image 420 qui supprime la zone centrale comportant le motif et les zones supérieure et inférieure de l'image. Sur les parties restantes est réalisé un calcul d'un cercle RMS du tour de l'iris à partir des extrémités droites des segments à gauche de l'image et des extrémités gauches des segments à droite de l'image 430. Pour ce calcul, les points trop éloignés du cercle RMS qui correspondent à des imperfections notamment causées par les cils ou la paupière sont rejetés à l'étape 440 et, pour les points restants, un nouveau cercle RMS est calculé pour suivre le contour de l'iris, ce cercle 93 est représenté en figure 9C sur l'image d'origine de l'œil.

La figure 10 représente un œil 80' regardant de biais et dont l'iris 82' est décalé par rapport au motif 83'. Pour cette position de l'œil, les transitions 84', 85' autour de zones sombres 86', 87' correspondant à des couleurs homogènes sont décalées latéralement dans la figure 11A alors que dans la figure 11B on constate que les arcs de cercles 89' et 90' correspondant au bord de l'iris restent détectables. L'application du procédé de suivi conduit là aussi à recréer le cercle RMS 93' qui va être repositionné sur l'image d'origine comme représenté en figure 11C. Les ruptures de film lacrymal détectées sont alors relocalisées selon l'étape 450 selon la position du cercle définissant le contour de l'iris. Ceci permet d'ancrer les ruptures du film lacrymal au contour de l'œil plutôt qu'à l'image.

Cette séquence est réalisée pour chaque image préférablement après l'analyse du motif en lignes décrit plus haut.

Comme dit plus haut ce procédé est ici appliqué au repositionnement des zones de rupture mais il peut aussi être utilisé pour d'autres types de détections et méthodes utilisant un suivi de la position d'un œil.

Selon un aspect de la demande, le dispositif peut comporter un déclencheur manuel qui arme le dispositif, le déclenchement de la séquence de prises de vues étant alors réalisé sur l'occurrence d'un évènement tel qu'une série de deux clignements de paupières du patient. Pour ce faire, le système comporte un procédé de reconnaissance de clignement de paupières qui permet de démarrer la séquence de mesure automatiquement. De même le système peut arrêter la séquence de mesures automatiquement sur détection d'un clignement de paupières ultérieur ou arrêter la séquence automatiquement après une temporisation, par exemple 15 secondes.

La séquence de prise de vues peut comporter de 30 à 50 images par exemple et dans le cas d'une séquence de prise de vues de 15 secondes avec prise de vues toutes les 0,3 secondes la séquence comporte 45 images. L'analyse des images peut se faire après la séquence de prise de vues et du fait de la solution choisie de travailler sur un motif fait de lignes, le temps de traitement reste réduit, par exemple de 15 secondes avec un ordinateur standard.

Lorsque la mesure est terminée, le praticien a à disposition, d'une part, une cartographie spatiale et temporelle des ruptures du film lacrymal au sein de la surface cornéenne et, d'autre part, une courbe temporelle retraçant l'apparition des ruptures du film lacrymal en fonction du temps. Cette courbe temporelle, et notamment sa pente, va révéler la cinétique d'apparition des ruptures du film lacrymal. Ceci permet d'affiner l'interprétation de l'examen pratiqué.

L'invention ne se limite pas aux exemples décrits ci-avant, seulement à titre d'exemple, mais elle englobe toutes les variantes comme une distribution ou une progression de la hauteur des lignes différentes que pourra envisager l'homme de l'art dans le cadre de la protection recherchée. En particulier, comme dit plus haut, les lignes du motif qui sont des lignes parallèles horizontales selon l'exemple représenté peuvent être remplacées par des lignes verticales, une rotation de l'image permettant par exemple d'appliquer les moyens de traitement d'image de détection des déformations des lignes à cette configuration sans en changer la direction.

## Revendications

1. Dispositif de détection d'une ou plusieurs ruptures d'un film lacrymal, comportant :
a. une plaque translucide munie d'une mire (10) destinée à être positionnée devant au moins un œil (101) d'un patient (100), la mire étant pourvue d'un motif (11) destiné à se réfléchir sur l'œil du patient,
b. au moins une caméra (21, 22) photographique numérique reliée à un système de calcul (30) pourvu de moyens de traitement et d'analyse d'images, un objectif de la caméra étant positionné pour pointer vers l'œil du patient afin de photographier une réflexion du motif (11) de la mire (10) sur l'œil du patient, où :
c. le motif de la mire (10) est pourvu d'une série de lignes sous forme d'une alternance de lignes transparentes (13) et de lignes opaques (12) horizontales ou verticales de formation, par rétroéclairage, de lignes claires et sombres réfléchies sur l'œil du patient,
d. les moyens de traitement et d'analyse d'images sont configurés pour détecter des déformations desdites lignes claires ou sombres réfléchies sur l'œil du patient et pour identifier les ruptures de film lacrymal révélées par ces déformations.

2. Dispositif selon la revendication 1, pour lequel les lignes de la série de lignes sont des lignes parallèles.

3. Dispositif selon la revendication 1 ou 2, pour lequel la largeur des lignes est croissante depuis une ligne médiane de la mire vers des bords de la mire.

4. Dispositif selon l'une quelconque des revendications précédentes, pour lequel la mire (10a) est pourvue d'une courbure cylindrique selon une génératrice verticale pour former une portion de cylindre et suivre la courbure de la tête du patient (100).

5. Dispositif selon l'une quelconque des revendications précédentes, pour lequel les moyens de traitement et d'analyse d'image comportent des moyens de conversion de l'image en niveaux de gris.

6. Dispositif selon l'une quelconque des revendications précédentes, pour lequel les moyens de traitement et d'analyse d'images comportent des moyens de filtrage passe-bande anisotropes.

7. Dispositif selon l'une quelconque des revendications précédentes, pour lequel les moyens de traitement et d'analyse d'images comportent des moyens d'analyse de l'image selon des successions de pixels perpendiculaires à la direction des lignes, des moyens de recherche de segments lumineux ou sombres dans lesdites successions de pixels, des moyens de quantification de la taille desdits segments lumineux ou sombres et d'élimination des segments dont la taille est incompatible avec une image des lignes du motif.

8. Dispositif selon la revendication 7, pour lequel les moyens de traitement et d'analyse d'images comportent des moyens de marquage/catalogage d'objets segments lumineux ou sombres adaptés à réaliser une reconstruction de premiers objets correspondant à des branches de lignes claires ou sombres du motif et à réaliser une élimination de seconds objets de forme non compatible avec lesdites lignes claires ou sombres.

9. Dispositif selon la revendication 8, pour lequel les moyens de traitement et d'analyse d'images comportent des moyens de calcul de raccordement de branches de lignes claires ou sombre sur un même axe, des moyens de calcul d'une régression polynomiale sur les données de lignes claires ou sombres en sorte de calculer des courbes RMS représentatives de bords desdites lignes et des moyens de calcul de détection de zones de rupture du film lacrymal sur des points d'image des bords de lignes dont la distance avec ladite courbe est au-delà d'une valeur de tolérance donnée.

10. Dispositif selon l'une quelconque des revendications précédentes, comportant des moyens de suivi de l'œil ou des yeux du patient basés sur une reconnaissance et un suivi d'iris en sorte de recaler les ruptures de film lacrymal détectées par rapport à l'œil analysé.

11. Dispositif selon l'une quelconque des revendications précédentes, pour lequel la plaque translucide rétroéclairée (10a) porteuse de la mire (10) et la ou les caméras (21, 22) sont intégrées dans un appareil de mesure ophtalmique (43, 44).

12. Dispositif selon l'une quelconque des revendications précédentes, pour lequel la mire (10) elle est réalisée au moyen d'un film polymère transparent pourvu de lignes opaques (12) imprimées ou sérigraphiées sur ledit film et séparées par des lignes transparentes (13), ledit film étant conformé pour se positionner sur une plaque translucide rétroéclairée (10a, 23) du dispositif.

13. Dispositif selon la revendication 12 pour lequel la mire comporte au moins une zone évidée (14) entourée d'un cadre opaque (15) ou des zones de substrat transparentes dans une zone médiane de la mire.

14. Procédé de détection de ruptures de film lacrymal au moyen d'un dispositif selon l'une quelconque des revendications précédentes, **caractérisé en ce qu'**il comporte une détection de clignement de paupière fournissant une origine des temps et au moins une séquence comportant une succession de prises d'images et de calcul de zones de rupture à partir de l'origine des temps et jusqu'à un clignement de paupière suivant.

15. Procédé de détection de ruptures de film lacrymal selon la revendication 14, comportant une prise d'image (200) toutes les 0,2 à 0,5 secondes et préférablement toutes les 0,3 secondes.

16. Procédé de détection de ruptures de film lacrymal selon la revendication 14 ou 15, comportant pour chaque image prise, une succession d'étapes de traitement et d'analyse comportant
- une conversion de l'image en niveaux de gris (205);
- un filtrage (210) de l'image convertie au moyen d'un filtre passe-bande anisotrope afin de réduire le vignettage et d'homogénéiser la luminosité de l'image ;
- une recherche de segments lumineux ou sombres colonne par colonne (220) dans l'image, une étape de quantification (230) de la taille desdits segments lumineux ou sombres et une étape d'élimination (235) des segments dont la taille est incompatible avec une correspondance avec des lignes du motif ;
- une étape de marquage/catalogage (240) d'objets segments lumineux ou segments sombres, de reconstruction (270) de premiers desdits objets correspondant à des objets branches de lignes du motif et une étape d'élimination (260) de seconds desdits objets de forme non compatible avec lesdites lignes du motif ;
- une étape de raccordement de branches de lignes de même niveau (280) et une étape de calcul d'une régression polynomiale (285) sur les données de lignes en sorte de calculer une courbe RMS des bords de lignes ;
- un calcul de zones de rupture du film lacrymal (290) par calcul de la distance des points de bords de lignes à ladite courbe, lesdites zones de rupture correspondant aux bords de lignes dont la distance à ladite courbe est supérieure à une valeur de tolérance donnée.

17. Procédé de détection de ruptures de film lacrymal selon l'une quelconque des revendications 14 à 16 comportant des étapes de suivi de l'œil ou des yeux du patient (400, 410, 420, 430, 440, 450) au moyen d'un procédé de suivi d'iris en sorte de repositionner les zones de rupture de film lacrymal détectées par rapport à l'œil analysé.

18. Procédé selon la revendication 17 pour lequel les étapes de suivi de l'œil comportent :
- une première étape de transformation de l'image par l'application d'un filtre passe bande anisotrope (400) appliqué dans la direction de la largeur de l'œil pour produire des paires de transition montantes sombre vers clair et descendantes clair vers sombre selon un axe horizontal de l'œil ;
- une étape de segmentation de l'image (410) pour rechercher les paires de transitions montante et descendante (88, 89, 90, 91) qui forment des segments devant être représentatifs de zones lumineuses dans l'image ;
- une étape de filtrage de l'image (420) qui élimine les segments clairs de la zone centrale comportant le motif et des zones supérieure et inférieure de l'image ;
- une étape de prise en compte des segments clairs, les autres zones de l'image n'étant plus considérées dans cette analyse, et un premier calcul d'un cercle RMS du tour de l'iris (430) à partir des extrémités droites des segments clairs à gauche de l'image et des extrémités gauches des segments clairs à droite de l'image;
- une étape (440) de suppression des points trop éloignés du cercle RMS ;
- pour les points restants, une nouvelle étape de calcul de cercle RMS (93) pour suivre le contour de l'iris.

19. Programme informatique comportant des instructions qui conduisent le dispositif selon les revendications 1 à 13 à la mise en œuvre du procédé selon l'une des revendications 14 à 18 lorsque ce programme est exécuté par un processeur.

20. Support d'enregistrement non transitoire lisible par un ordinateur sur lequel est enregistré un programme comprenant des instructions qui conduisent le dispositif selon les revendications 1 à 13 à la mise en œuvre du procédé selon l'une des revendications 14 à 18 lorsque ce programme est exécuté par un processeur.

## Patentansprüche

1. Vorrichtung zum Erfassen eines oder mehrerer Tränenfilmrisse, umfassend:
a. eine lichtdurchlässige Platte mit einem Visier (10), dazu bestimmt, vor wenigstens einem Auge (101) eines Patienten (100) positioniert zu werden, wobei das Visier mit einem Muster (11) versehen ist, das dazu bestimmt ist, sich auf dem Auge des Patienten zu reflektieren,
b. wenigstens eine digitale Fotokamera (21, 22), die mit einem Rechnersystem (30) verbunden ist, das mit Mitteln zur Bildverarbeitung und -analyse ausgestattet ist, wobei ein Objektiv der Kamera dazu positioniert ist, auf das Auge des Patienten gerichtet zu sein, um eine Reflexion des Musters (11) des Visiers (10) auf dem Auge des Patienten zu fotografieren, wobei
c. das Muster des Visiers (10) mit einer Reihe von Linien in Form von abwechselnden transparenten (13) und undurchsichtigen (12) horizontalen oder vertikalen Linien versehen ist, die durch Hintergrundbeleuchtung helle und dunkle Linien bilden, die auf dem Auge des Patienten reflektiert werden,
d. die Bildverarbeitungs- und -analysemittel dazu ausgebildet sind, Verformungen der auf das Auge des Patienten reflektierten hellen oder dunklen Linien zu erkennen und durch diese Verformungen offensichtlich gewordene Tränenfilmrisse zu identifizieren.

2. Vorrichtung nach Anspruch 1, wobei die Linien der Linienreihe parallele Linien sind.

3. Vorrichtung nach Anspruch 1 oder 2, wobei die Breite der Linien von einer Mittellinie des Visiers zu den Rändern des Visiers hin zunimmt.

4. Vorrichtung nach einem der vorhergehenden Ansprüche, wobei das Visier (10 a) mit einer zylindrischen Krümmung entlang einer vertikalen Erzeugenden versehen ist, um einen Zylinderabschnitt zu bilden und der Krümmung des Kopfes des Patienten (100) zu folgen.

5. Vorrichtung nach einem der vorhergehenden Ansprüche, wobei die Bildverarbeitungs- und -analysemittel Mittel zum Umwandeln des Bildes in Graustufen umfassen.

6. Vorrichtung nach einem der vorhergehenden Ansprüche, wobei die Bildverarbeitungs- und -analysemittel anisotrope Bandpassfiltermittel umfassen.

7. Vorrichtung nach einem der vorhergehenden Ansprüche, wobei die Bildverarbeitungs- und -analysemittel Mittel zur Analyse des Bildes nach einer Abfolge von Pixeln senkrecht zur Richtung der Linien umfassen sowie Mittel zur Suche nach hellen oder dunklen Segmenten in den genannten Abfolgen von Pixeln, Mittel zur Quantifizierung der Größe der hellen oder dunklen Segmente und zur Eliminierung der Segmente, deren Größe mit einem Bild der Linien des Musters unvereinbar ist.

8. Vorrichtung nach Anspruch 7, wobei die Bildverarbeitungs- und -analysemittel Mittel zum Markieren/Katalogisieren heller oder dunkler Objektsegmente umfassen, die dazu geeignet sind, eine Rekonstruktion erster Objekte, die hellen oder dunklen Linienabschnitten des Musters entsprechen, durchzuführen und zweite Objekte zu eliminieren, deren Form mit den hellen oder dunklen Linien nicht kompatibel ist.

9. Vorrichtung nach Anspruch 8, wobei die Bildverarbeitungs- und -analysemittel Mittel zum Berechnen der Verbindung von hellen oder dunklen Linienabschnitten auf derselben Achse, Mittel zur Berechnung einer polynomischen Regression der hellen oder dunklen Liniendaten, um RMS-Kurven zu berechnen, die die Ränder der Linien repräsentieren, und Mittel zur Berechnung der Erkennung von Tränenfilmunterbrechungsbereichen an Bildpunkten der Linienrändern, deren Abstand zu der Kurve einen vorgegebenen Toleranzwert überschreitet, umfassen.

10. Vorrichtung nach einem der vorhergehenden Ansprüche, umfassend Mittel zur Verfolgung des Auges oder der Augen des Patienten auf der Grundlage einer Iriserkennung und -verfolgung, um die erkannten Tränenfilmrisse in Bezug auf das analysierte Auge neu zu positionieren.

11. Vorrichtung nach einem der vorhergehenden Ansprüche, wobei die hinterleuchtete lichtdurchlässige Platte (10a), die das Visier (10) trägt, und die Kamera(s) (21, 22) in ein ophthalmologisches Messgerät (43, 44) integriert sind.

12. Vorrichtung nach einem der vorhergehenden Ansprüche, wobei das Visier (10) aus einer transparenten Polymerfolie besteht, die mit undurchsichtigen Linien (12) bedruckt oder siebgedruckt ist, die durch transparente Linien (13) voneinander getrennt sind, wobei die Folie so geformt ist, dass sie auf einer hinterleuchteten durchscheinenden Platte (10a, 23) der Vorrichtung positioniert werden kann.

13. Vorrichtung nach Anspruch 12, wobei das Visier wenigstens einen ausgesparten Bereich (14) aufweist, der von einem undurchsichtigen Rahmen (15) oder transparenten Substratbereichen in einem mittleren Bereich des Visiers umgeben ist.

14. Verfahren zum Erkennen von Tränenfilmrissen mittels einer Vorrichtung nach einem der vorhergehenden Ansprüche, **dadurch gekennzeichnet, dass** sie eine Blinzelerkennung umfasst, die einen Zeitursprung liefert, und wenigstens eine Sequenz, die eine Abfolge von Bildaufnahmen und Berechnungen von Bruchstellen vom Zeitursprung bis zu einem nächsten Blinzeln umfasst.

15. Verfahren zur Erkennung von Tränenfilmrissen nach Anspruch 14, umfassend eine Bildaufnahme (200) alle 0,2 bis 0,5 Sekunden und bevorzugt alle 0,3 Sekunden.

16. Verfahren zur Erkennung von Tränenfilmrissen nach Anspruch 14 oder 15, das für jedes aufgenommene Bild eine Abfolge von Verarbeitungsschritten und Analyseschritten umfasst, die Folgendes umfassen
- eine Umwandlung des Bildes in Graustufen (205);
- ein Filtern (210) des umgewandelten Bildes mittels eines anisotropen Bandpassfilters, um die Vignettierung zu reduzieren und die Helligkeit des Bildes zu homogenisieren;
- eine Suche nach hellen oder dunklen Segmenten Spalte für Spalte (220) im Bild, einen Quantisierungsschritt (230) der Größe der hellen oder dunklen Segmente und einen Schritt zum Eliminieren (235) der Segmente, deren Größe mit einer Übereinstimmung mit Linien des Musters unvereinbar ist;
- einen Schritt zum Markieren/Katalogisieren (240) heller oder dunkler Segmentobjekte, zum Rekonstruieren (270) erster dieser Objekte, die Linienabschnittsobjekten des Musters entsprechen, und einen Schritt zum Eliminieren (260) zweiter dieser Objekte, deren Form nicht mit den Linien des Musters kompatibel ist;
- einen Schritt zum Verbinden von Linienabschnitten derselben Ebene (280) und einen Schritt zum Berechnen einer polynomischen Regression (285) der Liniendaten, um eine RMS-Kurve der Linienränder zu berechnen;
- eine Berechnung von Tränenfilm-Bruchstellen (290) durch Berechnung des Abstands der Linienrandpunkte zu der Kurve, wobei die Bruchstellen den Linienrändern entsprechen, deren Abstand zu der Kurve größer als ein vorgegebener Toleranzwert ist.

17. Verfahren zum Erkennen von Tränenfilmrissen nach einem der Ansprüche 14 bis 16, das Schritte zum Verfolgen des Auges oder der Augen des Patienten (400, 410, 420, 430, 440, 450) mittels eines Irisverfolgungsverfahrens umfasst, um die erkannten Tränenfilmrisse in Bezug auf das analysierte Auge neu zu positionieren.

18. Verfahren nach Anspruch 17, wobei die Schritte zum Verfolgen des Auges umfassen:
- einen ersten Schritt des Transformierens des Bildes durch Anwenden eines anisotropen Bandpassfilters (400), der in Richtung der Breite des Auges angewendet wird, um aufsteigende Übergangspaare von dunkel nach hell und absteigende Übergangspaare von hell nach dunkel entlang einer horizontalen Achse des Auges zu erzeugen;
- einen Bildsegmentierungsschritt (410) zum Suchen der aufsteigenden und absteigenden Übergangspaare (88, 89, 90, 91), die Segmente bilden, die für helle Bereiche im Bild repräsentativ sein sollen;
- einen Bildfilterungsschritt (420), der die hellen Segmente aus dem mittleren Bereich, der das Muster enthält, und aus den oberen und unteren Bereichen des Bildes entfernt;
- einen Schritt zum Berücksichtigen der hellen Segmente, wobei die anderen Bereiche des Bildes in dieser Analyse nicht mehr berücksichtigt werden, und zum ersten Berechnen eines RMS-Kreises um die Iris (430) aus den rechten Enden der hellen Segmente links im Bild und den linken Enden der hellen Segmente rechts im Bild;
- einen Schritt (440) zum Löschen der Punkte, die zu weit vom RMS-Kreis entfernt sind;
- für die verbleibenden Punkte einen neuen Schritt zur Berechnung des RMS-Kreises (93), um der Kontur der Iris zu folgen.

19. Computerprogramm, umfassend Anweisungen, die die Vorrichtung nach den Ansprüchen 1 bis 13 zur Durchführung des Verfahrens nach einem der Ansprüche 14 bis 18 veranlassen, wenn dieses Programm von einem Prozessor ausgeführt wird.

20. Nichtflüchtiger, computerlesbarer Speicher, auf dem ein Programm gespeichert ist, das Befehle enthält, die die Vorrichtung nach den Ansprüchen 1 bis 13 zur Durchführung des Verfahrens nach einem der Ansprüche 14 bis 18 veranlassen, wenn dieses Programm von einem Prozessor ausgeführt wird.

## Claims

1. Device for detecting one or more break-ups of a tear film, comprising:
a. a translucent plate equipped with a test chart (10) intended to be positioned in front of at least one eye (101) of a patient (100) and that is provided with a pattern (11) intended to be reflected from the eye of the patient,
b. at least one digital photographic camera (21, 22) connected to a computing system (30) provided with means for processing and analyzing images, an objective of the camera pointing toward the patient's eye in order to photograph a reflection of the pattern (11) of the test chart (10) from the patient's eye,
c. the pattern of the test chart (10) is provided with a series of lines taking the form of an alternation of horizontal or vertical transparent lines (13) and opaque lines (12) creating, by backlighting, light and dark lines reflected on the eye of the patient
d. the means for processing and analyzing images are configured to detect deformations of said light or dark lines of the pattern of the test chart reflected from the patient's eye and to identify tear-film break-ups revealed by these deformations.

2. Device as claimed in claim 1, wherein the lines of the series of lines are parallel lines.

3. Device as claimed in claim 1 or 2, wherein the width of the lines increases from a median line of the test chart toward the edges of the test chart.

4. Device as claimed in any one of the preceding claims, wherein the test chart (10a) is provided with a cylindrical curvature generated using a vertical generatrix, so that it forms a portion of a cylinder and follows the curvature of the head of the patient (100).

5. Device as claimed in any one of the preceding claims, wherein the means for processing and analyzing images comprise means for converting the image to grayscale.

6. Device as claimed in any one of the preceding claims, wherein the means for processing and analyzing images comprise anisotropic band-pass filtering means.

7. Device as claimed in any one of the preceding claims, wherein the means for processing and analyzing images comprise means for analyzing the image in successions of pixels perpendicular to the direction of the lines, means for searching for bright or dark segments in said successions of pixels, and means for quantifying the size of said bright or dark segments and for removing segments the size of which is incompatible with an image of the lines of the pattern.

8. Device as claimed in claim 7, wherein the means for processing and analyzing images comprise means for marking/cataloging bright or dark segment of objects, which means are suitable for reconstructing first objects corresponding to lengths of bright or dark lines of the pattern and for removing second objects of shape incompatible with said light or dark lines.

9. Device as claimed in claim 8, wherein the means for processing and analyzing images comprise computing means for joining lengths of light or dark lines on the same axis, means for computing a polynomial regression on the light- or dark-line data so as to compute RMS curves representative of edges of said lines and computing means for detecting regions of break-up of the tear film for image points of the line edges the distance of which to said curve is larger than a given tolerance value.

10. Device as claimed in any one of the preceding claims, comprising means for tracking the patient's eye or eyes based on iris recognition and tracking so as to align the detected tear-film break-ups with the analyzed eye.

11. Device as claimed in any one of the preceding claims, wherein the backlit translucent plate (10a) bearing the test chart (10) and the one or more cameras (21, 22) are integrated into an ophthalmic measuring apparatus (43, 44).

12. Device as claimed in any one of the preceding claims, wherein the test chart (10) is made of a transparent polymer film provided with opaque lines (12) that are printed or screen-printed on said film ans separated by transparent lines (13), said film being designed to be positioned on a backlit translucent plate of the device.

13. Device as claimed in claim 12, wherein the test chart comprising at least one a hollowed-out area (14) encircled by an opaque frame (15) or transparent substrate areas in a median region of the test chart,

14. Method for detecting tear-film break-ups by means of a device as claimed in any one of the preceding claims, **characterized in that** it comprises detecting an eyelid blink that delivers a start time, and performing at least one sequence comprising successively capturing images and computing regions of break-up from the start time to the next eyelid blink.

15. Method for detecting tear-film break-ups as claimed in claim 14, comprising capturing (200) an image every 0.2 to 0.5 seconds and preferably every 0.3 seconds.

16. Method for detecting tear-film break-ups as claimed in claim 14 or 15, comprising, for each captured image, a succession of processing and analyzing steps comprising
- converting (205) the image to grayscale;
- filtering (210) the converted image by means of an anisotropic band-pass filter in order to decrease vignetting and to increase the uniformity of the brightness of the image;
- searching (220) for bright or dark segments in the image column by column, a step (230) of quantifying the size of said bright or dark segments and a step (235) of removing segments the size of which is incompatible with a correspondence with lines of the pattern;
- a step (240) of marking/cataloging bright segments or dark segments of objects, of reconstructing (270) first of said objects corresponding to objects forming lengths of lines of the pattern, and a step (260) of removing second of said objects of shape incompatible with said lines of the pattern;
- a step (280) of joining lengths of lines of the same level, and a step (285) for computing a polynomial regression on the line data so as to compute an RMS curve of the line edges;
- computing (290) regions of break-up of the tear film by computing the distance of points of line edges to said curve, said regions of break-up corresponding to line edges the distance of which to said curve is larger than a given tolerance value.

17. Method for detecting tear-film break-ups as claimed in any one of claims 14 to 16, comprising steps (400, 410, 420, 430, 440, 450) of tracking the patient's eye or eyes by means of an iris-tracking method, so as to reposition the regions of break-up of the tear film that are detected with respect to the analyzed eye.

18. Method as claimed in claim 17, wherein the eye-tracking steps comprise:
- a first step (400) of transforming the image via application of an anisotropic band-pass filter that is applied in the direction of the width of the eye, to produce pairs of rising, dark to light, and falling, light to dark, transitions along a horizontal axis of the eye;
- a step (410) of segmenting the image to find the pairs of rising and falling transitions (88, 89, 90, 91), which form segments that are necessarily representative of bright regions in the image;
- a step (420) of image filtering, which removes light segments from the central region comprising the pattern and top and bottom regions of the image;
- a step (430) of taking into account light segments, the other regions of the image no longer being considered in this analysis, and computing a first time an RMS circle of the perimeter of the iris on the basis of the right ends of the light segments on the left of the image and of the left ends of the light segments on the right of the image;
- a step (440) of removing points that are too far from the RMS circle; and
- as regards the remaining points, a new step of computing an RMS circle (93) to follow the outline of the iris.

19. Computer program comprising instructions that cause the device according to claims 1 to 13 to execute the method as claimed in one of the claims 14 to 18 when this program is executed by a processor.

20. Computer-readable non-volatile storage medium on which is stored a program comprising instructions that cause the device according to claims 1 to 13 to implement the method as claimed in one of claims 14 to 18 when this program is executed by a processor.
